(19) Europäisches Patentamt
European Patent Office
Office européen des brevets

(11) EP 3 170 500 A1

(12) # EUROPEAN PATENT APPLICATION
published in accordance with Art. 153(4) EPC

(43) Date of publication:
**24.05.2017 Bulletin 2017/21**

(21) Application number: **15821760.4**

(22) Date of filing: **17.07.2015**

(51) Int Cl.:
***A61K 31/517*** (2006.01) ***A61K 9/06*** (2006.01)
***A61K 9/08*** (2006.01) ***A61K 9/16*** (2006.01)
***A61K 9/20*** (2006.01) ***A61K 9/48*** (2006.01)
***A61K 9/70*** (2006.01) ***A61K 31/519*** (2006.01)
***A61K 31/53*** (2006.01) ***A61P 27/02*** (2006.01)
***A61P 27/06*** (2006.01)

(86) International application number:
**PCT/JP2015/070477**

(87) International publication number:
**WO 2016/010130 (21.01.2016 Gazette 2016/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **17.07.2014 JP 2014146573**

(71) Applicant: **Santen Pharmaceutical Co., Ltd**
**Osaka-shi, Osaka 533-8651 (JP)**

(72) Inventor: **YOSHIDA, Atsushi**
**Ikoma-shi**
**Nara 630-0101 (JP)**

(74) Representative: **Müller-Boré & Partner**
**Patentanwälte PartG mbB**
**Friedenheimer Brücke 21**
**80639 München (DE)**

(54) **PROPHYLACTIC OR THERAPEUTIC AGENT FOR DISEASES OF POSTERIOR SEGMENT OF EYE**

(57) The present invention relates to a prophylactic or therapeutic agent for a posterior ocular disease, which comprises a compound represented by formula (1) below:

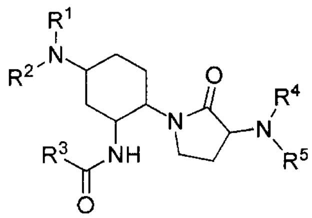

(1)

its enantiomer or diastereomer, or a pharmaceutically acceptable salt thereof (hereinbelow also referred to as the "present compound") as an active ingredient.

EP 3 170 500 A1

**Description**

[Technical Field]

The present invention relates to a prophylactic or therapeutic agent for a posterior ocular disease, which comprises, as an active ingredient, a 3-amino-2-oxopyrrolidine derivative, especially a compound represented by formula (1) below:

[Chem. 1]

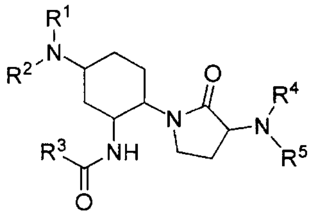

(1) ,

its enantiomer or diastereomer, or a pharmaceutically acceptable salt thereof (hereinbelow also referred to as the "present compound").

[Background Art]

The posterior ocular disease generally means a disease at the vitreous body, the retina, the choroid, the sclera or the optic nerve, and these diseases are deeply involved with neovascular expression. That is, in the posterior ocular diseases such as age-related macular degeneration, diabetic retinopathy, diabetic macular edema, retinal vein occlusion, uveitis, etc., it has been known that enhancement of neovascular expression is a main factor of formation of the pathological condition and progress of the pathological condition, so that it is useful for the treatment of these diseases to inhibit neovascularization (Non Patent Literature 1 and Non Patent Literature 2).

On the other hand, the compound represented by formula (1b) below:

[Chem. 2]

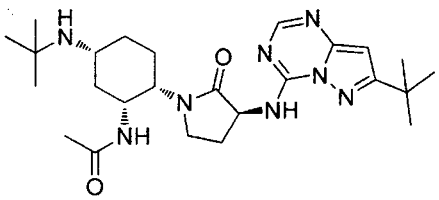

(1b)

has been described to have an inhibitory activity against CCR-2 and CCR-5 receptors, and it has been shown that this compound is effective for the treatment of inflammatory diseases, allergic diseases, autoimmune diseases, cancers and/or cardiovascular diseases (Patent Literature 1).

However, with respect to a 3-amino-2-oxopyrrolidine derivative, especially a compound represented by formula (1) below:

[Chem. 3]

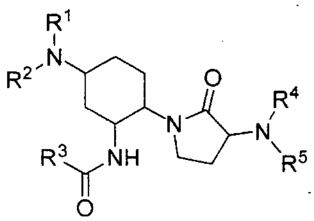

(1)

,

its enantiomer or diastereomer, or a pharmaceutically acceptable salt thereof (present compound), there is no report on

the study of its prophylactic or therapeutic effect against a posterior ocular disease.

[Citation List]

[Patent Literature]

[Patent Literature 1] International Publication No. WO 2011/046916

[Non Patent Literature]

[Non Patent Literature 1] Journal of Japanese Ophthalmological Society, 103, pp. 923-947 (1999)
[Non Patent Literature 2] New Illustrated Handbook of Clinical Ophthalmology, vol. 5 "Vitreoretinal disease", pp. 184-189, 232-237 (2000)

[Summary of Invention]

[Technical Problem]

An object of the present invention is to provide a prophylactic or therapeutic agent for a posterior ocular disease, which comprises a 3-amino-2-oxopyrrolidine derivative as an active ingredient.

[Solution to Problem]

The present inventors have intensively studied to search a novel prophylactic or therapeutic agent for a posterior ocular disease containing a 3-amino-2-oxopyrrolidine derivative as an active ingredient and, as a result, they have found that a compound represented by formula (1) below:

[Chem. 4]

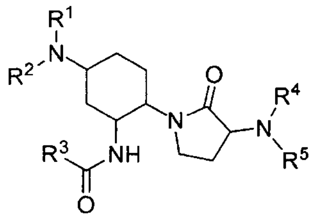

(1) ,

its enantiomer or diastereomer, or a pharmaceutically acceptable salt thereof has excellent inhibitory activity on angiogenesis and suppressive activity on vascular hyperpermeability at the posterior ocular tissue, such as the retina and the choroid, i.e., the present compound has excellent prophylactic or therapeutic effect against a posterior ocular disease, whereby they have accomplished the present invention.

That is, the present invention relates to the following.

The present invention relates to a prophylactic or therapeutic agent for a posterior ocular disease, which comprises a compound represented by formula (1) below:

[Chem. 5]

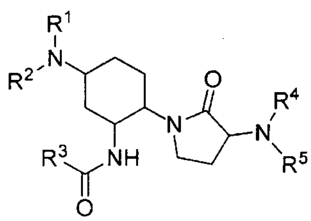

(1)

wherein,

$R^1$ and $R^2$ may be the same or different and represents a hydrogen atom, a lower alkyl group or a lower alkyl group substituted with a halogen atom;
$R^3$ represents a hydrogen atom, a halogen atom, a lower alkyl group or a lower alkyl group substituted with a halogen atom;
$R^4$ represents a hydrogen atom, a lower alkyl group or a lower alkyl group substituted with a halogen atom;
$R^5$ represents a nitrogen-containing bicyclic aromatic group which is unsubstituted or substituted with $R^6$; and
$R^6$ represents a halogen atom, a lower alkyl group or a lower alkyl group substituted with a halogen atom,

its enantiomer or diastereomer, or a pharmaceutically acceptable salt thereof as an active ingredient.

Another embodiment of the present invention relates to the prophylactic or therapeutic agent for a posterior ocular disease comprising the compound represented by formula (1) above, its enantiomer or diastereomer, or a pharmaceutically acceptable salt thereof as an active ingredient, wherein, in formula (1),

$R^1$ and $R^2$ may be the same or different and represents a hydrogen atom or a lower alkyl group;
$R^3$ represents a hydrogen atom or a lower alkyl group;
$R^4$ represents a hydrogen atom or a lower alkyl group;
$R^5$ represents a group represented by formula (2a), (2b) or (2c) below:

[Chem. 6]

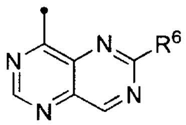
(2a)

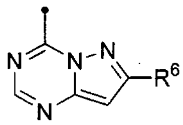
(2b)

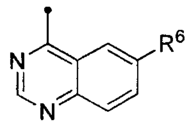
(2c) ;

and
$R^6$ represents a lower alkyl group or a lower alkyl group substituted with a halogen atom.

In this connection, the symbol "•" in each of formulae (2a), (2b) and (2c) represents a bonding point in which the group of one of these formulae as $R^5$ is bonded to the nitrogen atom in formula (1).

Another embodiment of the present invention relates to the prophylactic or therapeutic agent for a posterior ocular disease comprising the compound represented by formula (1) above, its enantiomer or diastereomer, or a pharmaceutically acceptable salt thereof as an active ingredient, wherein the compound represented by formula (1) is represented by formula (1') below:

[Chem. 7]

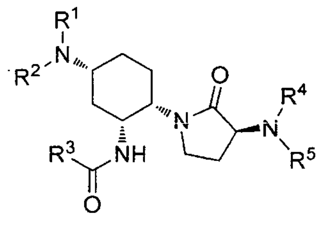
(1')

Another embodiment of the present invention relates to the prophylactic or therapeutic agent for a posterior ocular disease comprising the compound represented by formula (1) or (1') above, its enantiomer or diastereomer, or a pharmaceutically acceptable salt thereof as an active ingredient, wherein, in formula (1) or (1'),

$R^1$ represents a hydrogen atom;
$R^2$ represents a tert-butyl;
$R^3$ represents a methyl;
$R^4$ represents a hydrogen atom;
$R^5$ represents a group represented by formula (2b) below:

[Chem. 8]

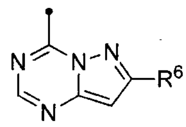

(2b) ;

and

$R^6$ represents a tert-butyl.

In this connection, the symbol "•" in formula (2b) represents a bonding point in which the group of this formula as $R^5$ is bonded to the nitrogen atom in formula (1).

Another embodiment of the present invention relates to the prophylactic or therapeutic agent for a posterior ocular disease comprising the compound represented by formula (1) above, its enantiomer or diastereomer, or a pharmaceutically acceptable salt thereof as an active ingredient, wherein the posterior ocular disease is a disease at a vitreous body, a retina, a choroid, a sclera or an optic nerve.

Another embodiment of the present invention relates to the prophylactic or therapeutic agent for a posterior ocular disease comprising the compound represented by formula (1) above, its enantiomer or diastereomer, or a pharmaceutically acceptable salt thereof as an active ingredient, wherein the posterior ocular disease is at least one member selected from the group consisting of age-related macular degeneration, diabetic retinopathy, diabetic macular edema, retinal pigmentary degeneration, proliferative vitreoretinopathy, retinal artery occlusion, retinal vein occlusion, uveitis, Leber's disease, retinopathy of prematurity, retinal detachment, retinal pigment epithelial detachment, central serous chorioretinopathy, central exudative chorioretinopathy, polypoidal choroidal vasculopathy, multiple choroiditis, neovascular maculopathy, retinal aneurysm, retinal angiomatous proliferation, ophthalmic nerve disorder caused by these diseases, ophthalmic nerve disorder caused by glaucoma and ischemic ophthalmic nerve disorder.

Another embodiment of the present invention relates to the prophylactic or therapeutic agent for a posterior ocular disease comprising the compound represented by formula (1) above, its enantiomer or diastereomer, or a pharmaceutically acceptable salt thereof as an active ingredient, wherein the posterior ocular disease is at least one member selected from the group consisting of age-related macular degeneration, diabetic retinopathy, diabetic macular edema, retinal vein occlusion and uveitis.

Another embodiment of the present invention relates to a choroidal neovascularization inhibiting agent comprising the compound represented by formula (1) above, its enantiomer or diastereomer, or a pharmaceutically acceptable salt thereof as an active ingredient.

Another embodiment of the present invention relates to the prophylactic or therapeutic agent for a posterior ocular disease or choroidal neovascularization inhibiting agent comprising the compound represented by formula (1) above, its enantiomer or diastereomer, or a pharmaceutically acceptable salt thereof as an active ingredient, wherein an administration form is instillation administration, intravitreal administration, subconjunctival administration, administration to the interior of the conjunctival sac, administration under the Tenon's capsule or oral administration.

Another embodiment of the present invention relates to the prophylactic or therapeutic agent for a posterior ocular disease or choroidal neovascularization inhibiting agent comprising the compound represented by formula (1) above, its enantiomer or diastereomer, or a pharmaceutically acceptable salt thereof as an active ingredient, wherein a dosage form is an eye drop, an ophthalmic ointment, an insert preparation, a plaster, an injection, a tablet, fine granules or a capsule.

In addition, the present invention also relates to the following.

Another embodiment of the present invention relates to use of the compound represented by formula (1) above, its enantiomer or diastereomer, or a pharmaceutically acceptable salt thereof in the prophylaxis or treatment for a posterior ocular disease.

Another embodiment of the present invention relates to use of the compound represented by formula (1) above, its enantiomer or diastereomer, or a pharmaceutically acceptable salt thereof for the manufacture of a medicine of a prophylaxis or treatment for a posterior ocular disease.

Another embodiment of the present invention relates to a pharmaceutical composition for a prophylaxis or treatment of a posterior ocular disease comprising a therapeutically effective amount of the compound represented by formula (1) above, its enantiomer or diastereomer, or a pharmaceutically acceptable salt thereof and an additive.

Another embodiment of the present invention relates to a method for prophylaxis or treatment of a posterior ocular disease, which method comprises administering an effective amount of the compound represented by formula (1) above, its enantiomer or diastereomer, or a pharmaceutically acceptable salt thereof.

[Advantageous Effects of Invention]

According to the present invention, a prophylactic or therapeutic agent for a posterior ocular disease comprising, as an active ingredient, the compound represented by formula (1) above, its enantiomer or diastereomer, or a pharmaceutically acceptable salt thereof can be provided.

[Description of Embodiments]

In the following, the present invention is explained in detail.
The term "halogen atom" refers to a fluorine atom, a chlorine atom, a bromine atom or an iodine atom.
The term "lower alkyl group" refers to a linear or branched $C_1$-$C_8$ alkyl group, preferably a linear or branched $C_1$-$C_6$ alkyl group. Specific examples of the lower alkyl group include methyl, ethyl, n-propyl, n-butyl, n-pentyl, n-hexyl, n-heptyl, n-octyl, isopropyl, isobutyl, sec-butyl, tert-butyl, isopentyl and the like.
The term "lower alkyl group substituted with a halogen atom" refers to a lower alkyl group substituted with one or more (for example, 2 or 3) halogen atoms, preferably a lower alkyl group substituted with 3 halogen atoms. As a specific example, there can be mentioned trifluoromethyl or the like. If a plurality of halogen atoms are present, the halogen atoms may be the same or different.
The term "nitrogen-containing bicyclic aromatic group" refers to a bicyclic aromatic ring in which at least one of the ring atoms is a nitrogen atom. For example, an aromatic ring having 8, 9 or 10 ring atoms including 1, 2, 3 or 4 nitrogen atom(s) is preferred. Specific examples include quinolinyl, isoquinolinyl, cinnolinyl, pyrazolo[1,5-a]pyridyl, imidazo[1,2-a]pyridyl, quinoxalinyl, quinazoline, benzotriazolyl, indolyl, indazolyl, pyrazolo[1,5-a][1,3,5]triazine, pyrimido[5,4-d]pyrimidine and the like.
The compound contained in the prophylactic or therapeutic agent for a posterior ocular disease of the present invention can be prepared according to the usual manner in the field of the organic synthetic chemistry. For example, it can be prepared according to the method disclosed in WO 2011/046916A, etc. In addition, a geometric isomer (cis-trans isomers), an optical isomer (an enantiomer, a diastereomer) or a tautomer of the compound can be isolated and purified by the usual manner, such as column chromatography, HPLC, etc.
The compound contained in the prophylactic or therapeutic agent for a posterior ocular disease of the present invention is a compound represented by formula (1) below:

[Chem. 9]

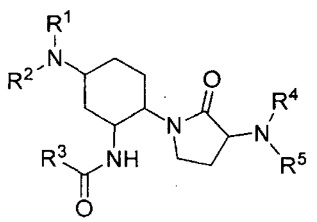

(1) ,

its enantiomer or diastereomer, or their pharmaceutically acceptable salts.
When a geometric isomer (cis-trans isomers), an optical isomer (an enantiomer, a diastereomer) or a tautomer of the compound represented by formula (1) above is present, they also fall within the scope of the compound represented by formula (1). In addition, the compound represented by formula (1) may be a mixture of one or two or more isomers selected from the group consisting of the geometric isomer (the cis-trans isomers), the optical isomer (the enantiomer, the diastereomer) and the tautomer.
In formula (1) above, $R^1$ and $R^2$ may be the same or different and represents a hydrogen atom, a lower alkyl group or a lower alkyl group substituted with a halogen atom.
In formula (1) above, $R^3$ represents a hydrogen atom, a halogen atom, a lower alkyl group or a lower alkyl group substituted with a halogen atom.
In formula (1) above, $R^4$ represents a hydrogen atom, a lower alkyl group or a lower alkyl group substituted with a halogen atom.
In formula (1) above, $R^5$ represents a nitrogen-containing bicyclic aromatic group which is unsubstituted or substituted with $R^6$.
In formula (1) above, $R^6$ represents a halogen atom, a lower alkyl group or a lower alkyl group substituted with a halogen atom,
**[0041]** Preferred examples of each of these substituents are given below.
In formula (1) above, preferably, $R^1$ and $R^2$ may be the same or different and is a hydrogen atom or a lower

alkyl group, more preferably, $R^1$ is a hydrogen atom and $R^2$ is a lower alkyl group, especially preferably, $R^1$ is a hydrogen atom and $R^2$ is a tert-butyl group.

In formula (1) above, $R^3$ is preferably a lower alkyl group, especially preferably a methyl group.

In formula (1) above, $R^4$ is preferably a hydrogen atom.

In formula (1) above, $R^5$ is preferably a group represented by formula (2a), (2b) or (2c) below:

[Chem. 10]

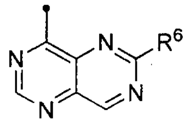
(2a)

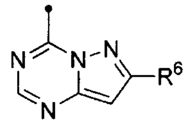
(2b)

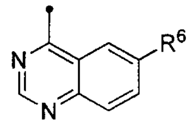
(2c)

,

especially preferably a group represented by formula (2b) below:

[Chem. 11]

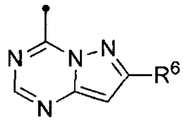
(2b)

In this connection, the symbol "•" in each of formulae (2a), (2b) and (2c) represents a bonding point in which the group of one of these formulae as $R^5$ is bonded to the nitrogen atom in formula (1).

$R^6$ is preferably a lower alkyl group or a lower alkyl group substituted with a halogen atom, more preferably a tert-butyl or a trifluoromethyl, most preferably a tert-butyl.

Formula (1) above is preferably formula (1') below:

[Chem. 12]

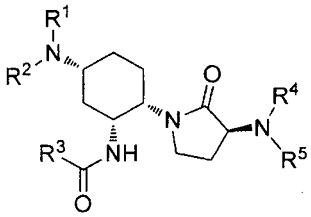
(1')

.

As a specific example of the compound represented by formula (1) or (1') above, there can be mentioned N-((-1R,2S,SR)-2-((S)-3-((6-(tert-butyl)pyrimido[5,4-d]pyrimidin-4-yl)amino)-2-oxopyrrolidin-1-yl)-5-(tert-butylamino)cyclohexyl)acetamide represented by formula (1a) below (hereinbelow also referred to as "compound 1a"):

[Chem. 13]

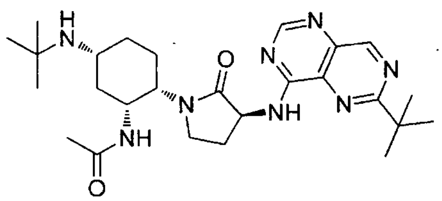
(1a)

,

enantiomer or diastereomer thereof, and a mixture thereof (such as racemic mixture and diastereomeric mixture).

As another specific example of the compound represented by formula (1) or (1') above, there can be mentioned

N-((1R,2S,5R)-5-(tert-butylamino)-2-((S)-3-(7-tert-butylpyrazolo[1,5-a][1,3,5]triazin-4-ylamino)-2-oxopyrrolidin-1-yl)cyclohexyl)acetamide represented by formula (1b) below (hereinbelow also referred to as "compound 1b"):

[Chem. 14]

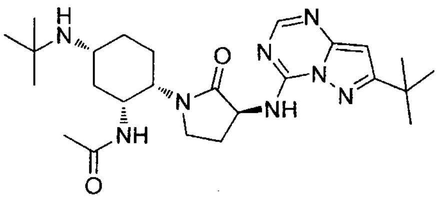

(1b)

,

enantiomer or diastereomer thereof, and a mixture thereof (such as racemic mixture and diastereomeric mixture).

As another specific example of the compound represented by formula (1) or (1') above, there can be mentioned N-((1R,2S,5R)-5-(tert-butylamino)-2-((S)-2-oxo-3-((6-(trifluoromethyl)quinazolin-4-yl)amino)pyrrolidin-1-yl)cyclohexyl)acetamide represented by formula (1c) below (hereinbelow also referred to as "compound 1c"):

[Chem. 15]

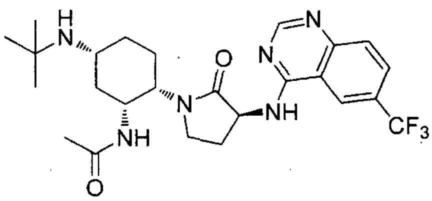

(1c)

,

enantiomer or diastereomer thereof, and a mixture thereof (such as racemic mixture and diastereomeric mixture).

As the "pharmaceutically acceptable salt" of the compound represented by formula (1), (1'), (1a), (1b) or (1c) above, there can be mentioned, for example, a salt with an inorganic acid or a salt with an organic acid. As the inorganic acids, there can be mentioned, for example, hydrochloric acid, hydrobromic acid, hydroiodic acid, nitric acid, sulfuric acid, phosphoric acid and the like. As the organic acids, there can be mentioned, for example, acetic acid, fumaric acid, maleic acid, succinic acid, citric acid, tartaric acid, adipic acid, gluconic acid, glucoheptonic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid and the like.

The compound represented by formula (1), (1'), (1a), (1b) or (1c) above may be in the form of a hydrate or a solvate.

When crystal polymorphism and a crystal polymorphism group (crystal polymorphism system) of the compound represented by formula (1), (1'), (1a), (1b) or (1c) above exist, these crystal polymorphs and crystal polymorphism group (crystal polymorphism system) also fall within the scope of the present compound. In this connection, the crystal polymorphism group (crystal polymorphism system) means a crystal form at the respective stages when the crystal form is variously changed by the conditions and states of preparation, crystallization, preservation, etc., of these crystals (incidentally, the state after preparation is also included in the above states), and the whole processes.

In the present invention, the posterior ocular disease means a disease at the vitreous body, the retina, the choroid, the sclera or the optic nerve. The posterior ocular disease is preferably at least one member selected from the group consisting of age-related macular degeneration (exudative age-related macular degeneration, atrophic age-related macular degeneration, early age-related macular degeneration), diabetic retinopathy, diabetic macular edema, retinal pigmentary degeneration, proliferative vitreoretinopathy, retinal artery occlusion, retinal vein occlusion, uveitis, Leber's disease, retinopathy of prematurity, retinal detachment, retinal pigment epithelial detachment, central serous chorioretinopathy, central exudative chorioretinopathy, polypoidal choroidal vasculopathy, multiple choroiditis, neovascular maculopathy, retinal aneurysm, retinal angiomatous proliferation, ophthalmic nerve disorder caused by these diseases, ophthalmic nerve disorder caused by glaucoma and ischemic ophthalmic nerve disorder, particularly preferably at least one member selected from the group consisting of age-related macular degeneration, diabetic retinopathy, diabetic macular edema, retinal vein occlusion and uveitis.

When the present compound is used for the treatment of the posterior ocular disease, it may be administered

to the patient orally or parenterally and, as the administration form, there may be mentioned oral administration, topical administration to eyes (instillation administration, administration to the interior of the conjunctival sac, intravitreal administration, subconjunctival administration, administration under the Tenon's capsule, etc.), intravenous administration, percutaneous administration, etc. As the preferred dosage form to be used for topically administering the present compound to eyes, eye drops or ophthalmic ointments or, alternatively, injections (in particular, a subconjunctival administration agent, a Tenon's capsule administration agent or an intravitreal administration agent) is used. The preparation containing the present compound as an active ingredient is formulated into a dosage form suitable for administration, together with a pharmaceutically acceptable additive(s), if necessary. As the dosage forms suitable for the oral administration, there may be mentioned, for example, tablets, capsules, granules, powders, etc. and, as the dosage forms suitable for parenteral administration, there may be mentioned, for example, injections, eye drops, ophthalmic ointments, plasters, gels, insert preparations, etc. These can be prepared using usual techniques generally used in the field of the art. Moreover, in order to take advantage of the sustained action of the therapeutic effect of the present invention more effectively, it may be made into a formulation with a construction as DDS, such as a preparation for intraocular implant, a microsphere or the like.

For example, a tablet can be prepared using an appropriately selected excipient, disintegrator, binder, lubricant, coating agent, corrigent and the like. As the excipient, there can be mentioned, for example, lactose, glucose, D-mannitol, anhydrous dibasic calcium phosphate, starch, sucrose and the like. As the disintegrator, there can be mentioned, for example, carboxymethyl cellulose, calcium carboxymethyl cellulose, croscarmellose sodium, crospovidone, starch, partially pregelatinized starch, low-substituted hydroxypropyl cellulose and the like. As the binder, there can be mentioned, for example, hydroxypropyl cellulose, ethyl cellulose, gum Arabic, starch, partially pregelatinized starch, polyvinyl pyrrolidone, polyvinyl alcohol and the like. As the lubricant, there can be mentioned, for example, magnesium stearate, calcium stearate, talc, hydrated silicon dioxide, hydrogenated oil and the like. As the coating agent, there can be mentioned, for example, refined white sugar, hydroxypropylmethyl cellulose, hydroxypropyl cellulose, methyl cellulose, ethyl cellulose, polyvinyl pyrrolidone and the like. As the corrigent, there can be mentioned, for example, citric acid, aspartame, ascorbic acid, menthol and the like.

For example, an injection can be prepared using an isotonicifier, a buffering agent, a surfactant, a thickener and the like selected depending on necessity. As the isotonicifier, there can be mentioned, for example, sodium chloride and the like. As the buffering agent, there can be mentioned, for example, sodium phosphate and the like. As the surfactant, there can be mentioned, for example, polyoxyethylene sorbitan monooleate and the like. As the thickener, there can be mentioned, for example, methyl cellulose and the like.

For example, an eye drop can be prepared using an isotonicifier, a buffering agent, a surfactant, a stabilizer, an anticeptic and the like selected depending on necessity. The pH of the eye drop may be within the range acceptable for an ophthalmic preparation and, usually, is preferably within the range of 4 to 8. As the isotonicifier, there can be mentioned, for example, sodium chloride, concentrated glycerin and the like. As the buffering agent, there can be mentioned, for example, sodium phosphate, sodium acetate and the like. As the surfactant, there can be mentioned, for example, polyoxyethylene sorbitan monooleate, polyoxyl 40 stearate, polyoxyethylene hydrogenated castor oil and the like. As the stabilizer, there can be mentioned, for example, sodium citrate, sodium edetate and the like. As the anticeptic, there can be mentioned, benzalkonium chloride, paraben and the like.

For example, an ophthalmic ointment can be prepared using a generally used base, such as white petrolatum, liquid paraffin and the like.

For example, an insert preparation can be prepared by pulverizing and mixing a biodegredable polymer, for example, hydroxypropyl cellulose, hydroxypropylmethyl cellulose, a carboxyvinyl polymer, a polyacrylic acid or the like with the present compound and compression-molding the resultant powder. If necessary, an excipient, a binder, a stabilizer and/or a pH adjuster may be used.

For example, a preparation for intraocular implant can be prepared using a biodegredable polymer, for example, a polylactic acid, a polyglycolic acid, a lactic acid-glycolic acid copolymer, hydroxypropyl cellulose and the like.

The dose of the present compound may be appropriately changed depending on a dosage form, the severity of the symptoms, age, body weight or volume of eye balls of the patient who receives the administration, judgment of a doctor or the like. In the case of the oral administration, generally 0.01 to 10,000 mg, preferably 0.1 to 5,000 mg, more preferably 0.5 to 2,500 mg of the present compound can be administered to an adult person at once or in several divided doses a day. In the case of the injection, generally 0.0001 to 2,000 mg of the present compound can be administered to an adult person at once or in several divided doses a day. In the case of the eye drops or insert preparation, the preparation having an active ingredient concentration of 0.000001 to 10% (w/v), preferably 0.00001 to 1% (w/v), more preferably 0.0001 to 0.1% (w/v) can be administered once or several times a day. In the case of the plasters, the plaster containing 0.0001 to 2,000 mg of the present compound can be applied to an adult person. In the case of the preparation for intraocular implant, the preparation for intraocular implant containing 0.0001 to 2,000 mg of the present compound can be implanted into the eyes of an adult person.

[Examples]

In the following, the results of Pharmacological tests and Preparation examples are shown, and these examples are intended to better understand the present invention and not to limit the scope of the present invention.

[Pharmacological test 1]

Usefulness of the present compounds was evaluated using a laser-induced rat choroidal neovascularization model (Invest. Ophthalmol. Vis. Sci., 40(2), 459-466 (1999)).

(Preparation method of krypton laser-induced rat choroidal neovascularization model animal)

A rat was generally anesthetized by intramuscular administration of 1 mL/kg of a mixed solution (7:1) comprising a 5% (W/V) ketamine hydrochloride injection solution and a 2% xylazine hydrochloride injection solution. 0.5% (W/V) Tropicamide-0.5% phenylephrine hydrochloride eye drop was dropped into eyes for mydriasis, and photocoagulation was carried out with a krypton laser photocoagulation apparatus. The photocoagulation was carried out at eight spots per eye scattered in the posterior part of eye ground, with the photocoagulation apparatus being focused on the deep retina and large retinal blood vessels being avoided (coagulation conditions: spot size 100 μm, output 100 mW, coagulation time 0.1 second). After the photocoagulation, ophthalmography was carried out to confirm laser irradiated sites.

(Test compound)

In the present pharmacological test, the above-mentioned compound 1b synthesized in accordance with the synthetic method described in WO 2011/046916A was used as the present compound.

(Drug administration method)

Compound 1b was mixed with a 1% (W/V) methyl cellulose solution (prepared by dissolving methyl cellulose in purified water) so that the concentration was 20 mg/mL and, from the surgery day of the photocoagulation, the resultant administration solution containing compound 1b was orally administered twice a day for 7 days (including the surgery day) at a dose of 100 mg/kg. To the base administered group, the 1% (W/V) methyl cellulose solution was administered in substantially the same manner.

(Evaluation method)

At the 7th day after the photocoagulation, the rat was generally anesthetized by intramuscular administration of 1 mL/kg of a mixed solution (7:1) comprising a 5% (W/V) ketamine hydrochloride injection solution and a 2% xylazine hydrochloride injection solution. 0.5% (W/V) Tropicamide-0.5% phenylephrine hydrochloride eye drop was dropped into eyes for mydriasis, and 0.1 mL of a 10% fluorescein solution was injected into a penile vein to carry out fluorescein fundus angiography. The spot of photocoagulation with no leakage of fluorescence detected by fluorescein fundus angiography was judged as negative (no neovascularization), and the spot of photocoagulation with leakage of fluorescence was judged as positive. The spot of photocoagulation with slight leakage of fluorescence was judged as positive (neovascularization exists) when 2 or more spots of such a state were detected in one eye. Thereafter, the rate of incidence of choroidal neovascularization (%) was calculated in accordance with Numerical Formula 1 below from the number of positive spot(s) relative to the eight spots of laser irradiation, and the suppressing rate (%) of the evaluated drug was calculated in accordance with Numerical Formula 2. The results of Compound 1b are shown in Table 1. Incidentally, a number of the samples of each administered group is 7 or 8.

[Numerical Formula 1]

$$\text{Rate of incidence of choroidal neovascularization \%} = (\text{Number of positive spot(s)} / \text{Total number of spots of photocoagulation}) \times 100$$

[Numerical Formula 2]

$$\text{Suppressing rate \%} = \{(A_0 - A_x) / A_0\} \times 100$$

$A_0$: Rate of incidence of choroidal neovascularization for the base administered group
$A_x$: Rate of incidence of choroidal neovascularization for the drug administered group

[Table 1]

| Group construction | Suppressing rate (%) |
|---|---|
| Compound 1b 100 mg/kg | 66.6 |

As apparent from Table 1, Compound 1b has been shown to inhibit choroidal neovascularization in a laser-induced rat choroidal neovascularization model animal. From these results, the present compound has been shown to have excellent prophylaxis or treatment effects against a posterior ocular disease.

[Preparation examples]

The drugs of the present invention are more specifically explained with reference to Preparation examples, but the present invention is not limited only to these Preparation examples.

Prescription example 1: Ophthalmic solution

In 100 ml

| | |
|---|---|
| Present compound: | 10 mg |
| Sodium chloride: | 900 mg |
| Polysorbate 80: | Appropriate amount |
| Disodium hydrogen phosphate: | Appropriate amount |
| Sodium dihydrogen phosphate: | Appropriate amount |
| Sterile purified water: | Appropriate amount |

The present compound and the other components mentioned above are added to sterile purified water, and the resultant mixture is thoroughly mixed to thereby prepare an eye drop. By appropriately changing the amount of the present compound to be added, an eye drop with a concentration of 0.05% (w/v) to 1% (w/v) can be prepared.

Prescription example 2: Ophthalmic ointment

In 100 g

| | |
|---|---|
| Present compound: | 0.3 g |
| Liquid paraffin: | 10.0 g |
| White petrolatum: | Appropriate amount |

The present compound is added to uniformly melt white petrolatum and liquid paraffin, and the resultant mixture is thoroughly mixed and gradually cooled to thereby prepare an ophthalmic ointment. By appropriately changing the amount of the present compound to be added, an ophthalmic ointment with a concentration of 0.05% (w/v) to 1% (w/w) can be prepared.

Prescription example 3: Tablet

In 100 mg

| | |
|---|---|
| Present compound: | 1 mg |
| Lactose: | 66.4 mg |
| Corn starch: | 20 mg |
| Calcium carboxymethylcellulose: | 6 mg |
| Hydroxypropylcellulose: | 6 mg |
| Magnesium stearate: | 0.6 mg |

To a mixture obtained by mixing the present compound and lactose in a mixer, calcium carboxymethylcellulose and hydroxypropylcellulose are added, and the resultant mixture is granulated. The obtained granules are dried and subjected to size regulation. Magnesium stearate is added to and mixed with the resultant size-regulated granules, and

the resultant mixture is subjected to tableting by a tableting machine. By appropriately changing the amount of the present compound, calcium carboxymethylcellulose and hydroxypropylcellulose to be added, a tablet with the content of the present compound of from 0.1 mg to 50 mg in 100 mg can be prepared.

| | |
|---|---|
| Prescription example 4: Injection or preparation for intravitreal administration | |
| In 10 ml | |
| Present compound: | 10 mg |
| Sodium chloride: | 90 mg |
| Polysorbate 80: | Appropriate amount |
| Sterile purified water: | Appropriate amount |

The present compound and the other components mentioned above are added to sterile purified water, and the resultant mixture is thoroughly mixed to dissolve or suspend to prepare an injection. By appropriately changing the amount of the present compound and the other components mentioned above to be added, an injection with a content of 2 mg to 200 mg of the present compound in 10 ml can be prepared. The thus prepared injection can be administered as an injection for ocular administration, for example, as a preparation for intravitreal administration.

## Claims

1. A prophylactic or therapeutic agent for a posterior ocular disease, which comprises a compound represented by formula (1) below:

[Chem. 16]

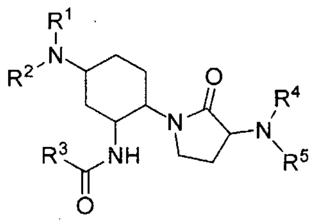

(1)

wherein,

$R^1$ and $R^2$ may be the same or different and represents a hydrogen atom, a lower alkyl group or a lower alkyl group substituted with a halogen atom;
$R^3$ represents a hydrogen atom, a halogen atom, a lower alkyl group or a lower alkyl group substituted with a halogen atom;
$R^4$ represents a hydrogen atom, a lower alkyl group or a lower alkyl group substituted with a halogen atom;
$R^5$ represents a nitrogen-containing bicyclic aromatic group which is unsubstituted or substituted with $R^6$; and
$R^6$ represents a halogen atom, a lower alkyl group or a lower alkyl group substituted with a halogen atom,

its enantiomer or diastereomer, or a pharmaceutically acceptable salt thereof as an active ingredient.

2. The prophylactic or therapeutic agent according to claim 1, wherein, in formula (1),

$R^1$ and $R^2$ may be the same or different and represents a hydrogen atom or a lower alkyl group;
$R^3$ represents a hydrogen atom or a lower alkyl group;
$R^4$ represents a hydrogen atom or a lower alkyl group;
$R^5$ represents a group represented by formula (2a), (2b) or (2c) below:

[Chem. 17]

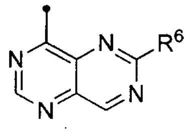
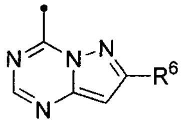
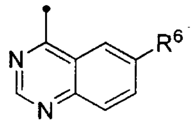

(2a) (2b) (2c) ;

and
$R^6$ represents a lower alkyl group or a lower alkyl group substituted with a halogen atom.

**3.** The prophylactic or therapeutic agent according to claim 1 or 2, wherein formula (1) represents formula (1') below:

[Chem. 18]

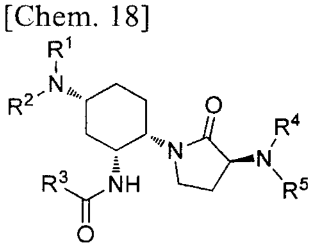

(1').

**4.** The prophylactic or therapeutic agent for a posterior ocular disease according to any one of claims 1 to 3, wherein, in formula (1) or (1'),

$R^1$ represents a hydrogen atom;
$R^2$ represents a tert-butyl;
$R^3$ represents a methyl;
$R^4$ represents a hydrogen atom;
$R^5$ represents a group represented by formula (2b) below:

[Chem. 19]

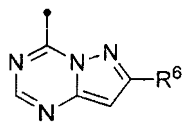

(2b) ;

and
$R^6$ represents a tert-butyl.

**5.** The prophylactic or therapeutic agent according to any one of claims 1 to 4, wherein the posterior ocular disease is a disease at a vitreous body, a retina, a choroid, a sclera or an optic nerve.

**6.** The prophylactic or therapeutic agent according to any one of claims 1 to 4, wherein the posterior ocular disease is at least one member selected from the group consisting of age-related macular degeneration, diabetic retinopathy, diabetic macular edema, retinal pigmentary degeneration, proliferative vitreoretinopathy, retinal artery occlusion, retinal vein occlusion, uveitis, Leber's disease, retinopathy of prematurity, retinal detachment, retinal pigment epithelial detachment, central serous chorioretinopathy, central exudative chorioretinopathy, polypoidal choroidal vasculopathy, multiple choroiditis, neovascular maculopathy, retinal aneurysm, retinal angiomatous proliferation, ophthalmic nerve disorder caused by these diseases, ophthalmic nerve disorder caused by glaucoma and ischemic ophthalmic nerve disorder.

**7.** The prophylactic or therapeutic agent according to claim 6, wherein the posterior ocular disease is at least one member selected from the group consisting of age-related macular degeneration, diabetic retinopathy, diabetic

macular edema, retinal vein occlusion and uveitis.

**8.** A choroidal neovascularization inhibiting agent comprising the compound represented by formula (1) above, its enantiomer or diastereomer, or a pharmaceutically acceptable salt thereof as an active ingredient.

**9.** The prophylactic or therapeutic agent or inhibiting agent according to any one of claims 1 to 8, wherein an administration form is instillation administration, intravitreal administration, subconjunctival administration, administration to the interior of the conjunctival sac, administration under the Tenon's capsule or oral administration.

**10.** The prophylactic or therapeutic agent or inhibiting agent according to any one of claims 1 to 9, wherein a dosage form is an eye drop, an ophthalmic ointment, an insert preparation, a plaster, an injection, a tablet, fine granules or a capsule.

**INTERNATIONAL SEARCH REPORT**

International application No. PCT/JP2015/070477

A. CLASSIFICATION OF SUBJECT MATTER
*A61K31/517*(2006.01)i, *A61K9/06*(2006.01)i, *A61K9/08*(2006.01)i, *A61K9/16*(2006.01)i, *A61K9/20*(2006.01)i, *A61K9/48*(2006.01)i, *A61K9/70*(2006.01)i, *A61K31/519*(2006.01)i, *A61K31/53*(2006.01)i, *A61P27/02*(2006.01)i,

According to International Patent Classification (IPC) or to both national classification and IPC

B. FIELDS SEARCHED

Minimum documentation searched (classification system followed by classification symbols)
A61K31/517, A61K9/06, A61K9/08, A61K9/16, A61K9/20, A61K9/48, A61K9/70, A61K31/519, A61K31/53, A61P27/02, A61P27/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY/MEDLINE/EMBASE/BIOSIS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2009-544756 A (Bristol-Myers Squibb Co.), 17 December 2009 (17.12.2009), claims; paragraph [0316] & US 2008/0027084 A1 & WO 2008/014360 A2 & EP 2046779 A1 & CN 101511822 A & KR 10-2009-0033915 A | 1-3,5-10 |
| Y | JP 2013-507444 A (Bristol-Myers Squibb Co.), 04 March 2013 (04.03.2013), claims; examples & US 2011/0086857 A1 & WO 2011/046916 A1 & EP 2488523 A1 & CN 102648201 A & KR 10-2012-0102052 A | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 August 2015 (04.08.15) | 11 August 2015 (11.08.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No. PCT/JP2015/070477

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2014-505040 A (Allergan, Inc.), 27 February 2014 (27.02.2014), paragraphs [0003] to [0005], [0068] to [0070] & US 2012/0157413 A1 & WO 2012/082566 A1 & EP 2651893 A1 & CN 103370326 A & KR 10-2013-0126960 A | 1-10 |
| Y | JP 2014-501245 A (Allergan, Inc.), 20 January 2014 (20.01.2014), paragraphs [0002] to [0005], [0059] to [0062] & US 2012/0157413 A1 & WO 2012/082568 A1 & EP 2651893 A1 & CN 103370326 A & KR 10-2013-0126960 A | 1-10 |
| Y | SAKURAI E et al., Targeted disruption of macrophage recruitment chemokines and chemokine receptors inhibits choroidal neovascularization., IOVS, Vol.45, No. Suppl.1, 2004, p.U804 | 1-10 |
| Y | Mihoko SUZUKI et al., "Myakuraku Maku Shinsei Kekkan ni Taisuru Ccr2 Antagonist no Yuyosei to Anzensei", Journal of Japanese Ophthalmological Society, 2012, vol.116, page 255 | 1-10 |
| Y | Motohiro KAMEI et al., "22.CCR2 Sogaizai ni yoru Myakuraku Maku Shinsei Kekkan Hassei Yokusei to Taishuku Koka", Momaku Myakuraku Maku·Shishinkei Ishukusho ni Kansuru Chosa Kenkyu, Heisei 24 Nendo Sokatsu·Buntan Kenkyu Hokokusho (3 Nen Keikaku no 2 Nenme), 2013, pages 69 to 72 | 1-10 |
| P,Y | JP 2014-193854 A (Santen Pharmaceutical Co., Ltd.), 09 October 2014 (09.10.2014), claims; examples & WO 2014/133072 A | 1-10 |

Form PCT/ISA/210 (continuation of second sheet) (July 2009)

**INTERNATIONAL SEARCH REPORT**

International application No.
PCT/JP2015/070477

Continuation of A. CLASSIFICATION OF SUBJECT MATTER
(International Patent Classification (IPC))

*A61P27/06*(2006.01)i

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011046916 A


### Non-patent literature cited in the description


- *Journal of Japanese Ophthalmological Society,* 1999, vol. 103, 923-947
- New Illustrated Handbook of Clinical Ophthalmology. Vitreoretinal disease. 2000, vol. 5, 184-189, 232-237
- *Invest. Ophthalmol. Vis. Sci.,* 1999, vol. 40 (2), 459-466